# EUROPÄISCHE PATENTSCHRIFT

(11) **EP 2 118 044 B1**
(45) Veröffentlichungstag und Bekanntmachung des Hinweises auf die Patenterteilung: **15.05.2013**
(21) Anmeldenummer: 07726909.0
(22) Anmeldetag: 14.03.2007
(51) Int. Cl.: C07C 29/80, C07C 31/20

(54) **VERFAHREN ZUR HERSTELLUNG VON SEHR REINEM 1,4-BUTANDIOL**
METHOD FOR THE PRODUCTION OF VERY PURE 1,4-BUTANEDIOL
PROCÉDÉ DE PRÉPARATION DE 1,4-BUTANEDIOL TRÈS PUR

(30) Priorität: 15.02.2007 EP 07102474
(43) Veröffentlichungstag der Anmeldung: 18.11.2009
(73) Patentinhaber: BASF SE, 67056 Ludwigshafen (DE)
(72) Erfinder: PINKOS, Rolf, 67098 Bad Dürkheim (DE); LORENZ, Rudolf Erich, 67245 Lambsheim (DE); BESTE, York Alexander, 67346 Speyer (DE)
(86) Internationale Anmeldenummer: PCT/EP2007/052418
(87) Internationale Veröffentlichungsnummer: WO 2008/098621

(56) Entgegenhaltungen:
- DD-A1- 200 465
- DE-A1- 2 055 892
- DE-A1- 10 021 703
- DE-A1- 19 801 089

## Beschreibung

Die Erfindung betrifft ein kontinuierliches Verfahren zur destillativen Reinigung von 1,4-Butandiol (BDO).

Die Herstellung von 1,4-Butandiol durch Umsetzung von Acetylen mit Formaldehyd und anschließende katalytische Hydrierung des erhaltenen 1,4-Butindiols ist eine seit vielen Jahren bekannte Reaktion.

Bei der Herstellung von 1,4-Butandiol durch Hydrierung von 1 ,4-Butindiol erhält man ein Rohprodukt, das neben 1,4-Butandiol, Wasser, Methanol, Propanol, Butanol, gamma-Butyrolacton, 2-Methyl-1,4-butandiol, 1,4-Butandiol, 2-(4-Hydroxybutoxy)-tetrahydofuran (im Folgenden Acetal genannt), Pentandiole wie z. B. 1,5-Pentandiol und 2-Methyl-1,5-Pentandiol, Salze, organische Hochsieder sowie weitere, mengenmäßig unbedeutende Nebenkomponenten enthalten kann.

Für die Gewinnung von reinem 1,4-Butandiol aus derartigen Rohprodukten ist aus DE-A 2 055 892 ein Verfahren zur destillativen Reinigung von rohem wasserhaltigen 1,4-Butandiol bekannt, bei dem das 1,4-Butandiol nach Abtrennung von Wasser und Leichtsiedern in zwei Destillationsstufen durch drei weitere Destillationskolonnen geleitet wird, wobei das Kopfprodukt der dritten Kolonne in eine vierte Kolonne geleitet wird, aus der reines 1,4-Butandiol als Sumpfprodukt gewonnen wird. Das Sumpfprodukt der dritten Kolonne, das 1,4-Butandiol geringer Reinheit und höher als 1,4-Butandiol siedende Bestandteile (Hochsieder) enthält, wird in eine fünfte Kolonne geführt, wo es in ein 1,4-Butandiol geringer Reinheit und Hochsieder getrennt wird. Das Butandiol geringerer Reinheit wird in die dritte Kolonne rückgeführt.

Die wasserhaltigen, durch Hydrierung von 1,4-Butindiol erhältlichen Rohprodukte werden durch das aus DE-A 2 055 892 bekannte Verfahren zwar aufgereinigt, um bei gängigen Weiterverarbeitungen des 1,4-Butandiols, beispielsweise zu Polyester- oder Polyurethanen, eingesetzt werden zu können, bei Anwendungen, bei denen die Anforderungen an das Butandiol jedoch besonders hoch sind, beispielsweise um besonders hohe Molgewichte bei Polyester zu bekommen, ist eine höhere Reinheit erforderlich.

Es stellte sich daher die Aufgabe, ein verbessertes Verfahren zu finden, welches wirtschaftlich die Aufreinigung von 1,4-Butandiol-Rohprodukten ermöglicht und das es erlaubt, ein für die Polyester bzw. Polyurethan-Herstellung besonders geeignetes Produkt herzustellen.

Diese Aufgabe wird gelöst durch ein Verfahren zur destillativen Reinigung von rohem, wasserhaltigen 1,4-Butandiol (1), bei dem man von leichter (niedriger) als 1,4-Butandiol siedenden Komponenten (Leichtsiedern) und Wasser befreites 1,4-Butandiol (5) durch drei Destillationskolonnen (III, IV, V) leitet, höher als 1,4-Butandiol siedende Komponenten (Hochsieder) aus dem Sumpf der ersten Kolonne (III) abzieht und in die Kolonne (V) (7) führt, 1,4-Butandiol vom Kopf der ersten Kolonne (III) (6) in die zweite Kolonne (IV) führt, das Sumpfprodukt der zweiten Kolonne (IV) (9) in die dritte Kolonne (V) führt, das Kopfprodukt der dritten Kolonne (V) (11) zumindest teilweise in die erste Kolonne (III) zurückführt, wobei in der zweiten Kolonne das molare Verhältnis von Sauerstoff zu 1,4-Butandiol kleiner als 1:500 ist, das dadurch gekennzeichnet ist, dass man aus einem Seitenabzug der zweiten Kolonne (IV) das reine 1,4-Butandiol (10) entnimmt.

Das erfindungsgemäße Verfahren ist auf rohes, wasserhaltiges 1,4-Butandiol, das durch unterschiedlichste Herstellungsverfahren erzeugt wurde, anwendbar. Besonders geeignet ist es für 1,4-Butandiol-Rohprodukte, die durch Hydrierung von 1,4-Butindiol entstanden sind. Es ermöglicht die Gewinnung eines hochreinen 1,4-Butandiols.

Nach dem neuen Verfahren nimmt man die destillative Reinigung des rohen, wasserhaltigen 1,4-Butandiols, das vorzugsweise durch Hydrierung von 1,4-Butindiol erhalten wurde, nach der an sich bekannten Abtrennung der Leichtsieder und des Wassers in den Kolonnen (I) und (II) in drei hintereinandergeschalteten Kolonnen (III, IV, V) so vor, wie es aus der Figur ersichtlich ist.

Der aus der Hydrierung des 1,4-Butindiols stammende Produktstrom wird üblicherweise direkt nach der Hydrierung in einen Abscheider geleitet, bei dem sich Gas- und Flüssigphase trennen. Die Gasphase enthält überwiegend Wasserstoff. Sofern mit Kreisgas hydriert wird, wird dieser Abscheider bevorzugt beim selben Druck betrieben wie die Hydrierung selbst, damit das daraus entnommene Gas nicht noch zusätzlich komprimiert werden muss. Ein Teil des Gasstroms kann als Abgas entsorgt werden.

Die flüssige Phase des Abscheiders kann über eine Druckentspannung in einen weiteren Gas-, Flüssig-Abscheider oder direkt in die Destillationseinheit geleitet werden. In beiden Fällen wird gelöstes Gas, überwiegend Wasserstoff, ausgeschleust und bevorzugt verbrannt, wobei Energie erzeugt werden kann.

Der Druck nach der Entspannung liegt im Allgemeinen zwischen Normaldruck und 20 bar, bevorzugt zwischen Normaldruck und 15 bar, besonders bevorzugt zwischen Normaldruck und 10 bar.

Der nach weitgehender Abtrennung des Wasserstoffs erhaltende Produktstrom der Hydrierung (1) enthält im Allgemeinen Methanol, Propanol, Butanol, Wasser, gamma-Butyrolacton, 2-Methyl-1,4-butandiol, 1,4-Butandiol, 2-(4-Hydroxybutoxy)-tetrahydofuran (im Folgenden Acetal genannt), Pentandiole wie z. B. 1,5-Pentandiol und 2-Methyl-1,5-Pentandiol, Salze, organische Hochsieder sowie weitere, mengenmäßig unbedeutende Nebenkomponenten.

Die Temperaturen der im Folgenden beschriebenen Destillationen werden durch die Dampfdrücke der in den Stoffströmen enthaltenen Komponenten und dem eingestellten Druck bestimmt. Die Destillationen laufen bevorzugt wärmeintegriert, um möglichst wenig Energie zu verbrauchen.

Zur destillativen Reinigung des rohen, wasserhaltigen 1,4-Butandiol's (1) werden bevorzugt mehrere Kolonnen als Destillationseinheiten verwendet. Unter Kolonnen oder Destillationseinheiten werden in der vorliegenden Anmeldung an sich bekannte Kolonnenarten wie beispielsweise als Packungskolonnen, Bodenkolonnen mit Siebböden, dual flow-Böden, Glöckenböden, Ventilböden ausgestattete Rektifikationskolonnen, Trennwandkolonnen oder Dünnschicht- oder Fallfilmverdampfer verstanden.

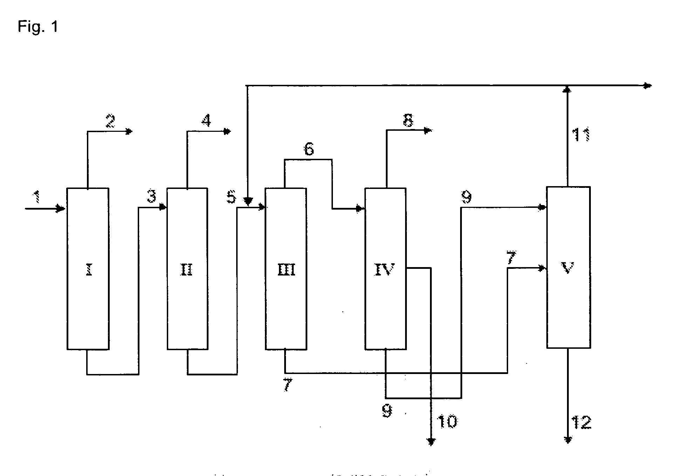

Leichtsieder wie Methanol, Propanol, Butanol und Wasser werden bei Drücken (absolut) zwischen 0,5 und 20 bar, bevorzugt zwischen 0,8 und 10 bar vom rohen, wasserhaltigen 1,4-Butandiol-haltigen Produktstrom (1) abgetrennt. Diese Abtrennung kann in mindestens einer Destillationskolonne erfolgen. Bevorzugt erfolgt die Abtrennung in mindestens zwei Destillationskolonnen, wobei in der ersten Kolonne (I) ein Gemisch aus Methanol, Propanol und Butanol, das auch Wasser enthält, abdestilliert wird. In einer weiteren Kolonne (II) oder mehreren weiteren Kolonnen, besonders bevorzugt zwei weiteren Kolonnen, die bevorzugt über eine Wärmeintegration verfügen, wird das verbliebene Wasser abdestilliert. Der Methanol, Propanol und Butanol sowie Wasser enthaltende Strom (2) kann entweder verbrannt, oder separat in die einzelnen Komponenten getrennt werden, um sie beispielsweise als Lösemittel in anderen Prozessen zu verwenden. Methanol kann beispielsweise in der Formaldehydherstellung verwendet werden. Bevorzugt wird der Strom 2 so aufgetrennt, dass in einer weiteren, in Figur 1 nicht gezeigten Kolonne, Methanol über Kopf abdestilliert wird, ein Gemisch aus Propanol, Butanol und Wasser über Seitenabzug abgetrennt wird. Das Gemisch wird bevorzugt so weit abgekühlt, dass es in zwei Phasen zerfällt, wobei die obere, die überwiegend Butanol und Propanol enthält, ausgeschleust und entweder weiter aufgetrennt oder verbrannt werden kann, während die untere Phase, die überwiegend Wasser enthält, ins Abwasser abgegeben oder in die Kolonne zurückgeführt wird und als Sumpfstrom überwiegend Wasser anfällt.

Das Sumpfprodukt (3) wird dann der Kolonne (II) zugeführt, aus der als Kopfprodukt (4) Wasser und als Sumpfprodukt (5) vorgereinigtes Rohbutandiol abgezogen werden kann.

Der nach der destillativen Abtrennung des Wassers und Leichtsiedern erhaltene Produktstrom (5) enthält neben bis zu 99,8 Gew.-% 1,4-Butandiol sowie gamma-Butyrolacton, 2-Methyl-1,4-butandiol, Acetal, Pentandiole, Salze, organische Hochsieder sowie weitere, mengenmäßig unbedeutende Nebenkomponenten und wird weiter destillativ aufgearbeitet.

Der Produktstrom (5) wird gemäß Figur 1 in der Kolonne (III) in flüchtige organische Bestandteile enthaltende Kopffraktion (6), die 90 bis 99,8 Gew.-% 1,4-Butandiol sowie gamma-Butyrolacton, 2-Methyl-1,4-butandiol, Acetal sowie weitere, Nebenkomponenten enthält wie Pentandiole, Hexandiole und Heptandiole und eine organische Hochsieder enthaltende Fraktion (7), aufgetrennt, die im Allgemeinen noch über 30 Gew.-% 1,4-Butandiol enthält. Dies wird üblicherweise bei einem Druck (absolut) von 0,005 bis 0,8 bar, bevorzugt zwischen 0,001 und 0,5 bar, besonders bevorzugt von 0,02 bis 0,2 bar durchgeführt. Die Sumpffraktion (7) wird einer weiteren Kolonne (V) zugeführt. Anstelle der Kolonne (III) kann auch ein Fallfilmverdampfer oder ein Dünnschichtverdampfer verwendet werden.

Kopffraktion (6) wird weiter destillativ in mindestens einer weiteren Kolonne (IV) in ein Kopfprodukt (8), eine Sumpffraktion (9) und einen Seitenstrom (10) aufgetrennt. Diese weitere Kolonne (IV) ist bevorzugt mindestens eine Rektifikationskolonne in Form einer Bodenkolonne mit Sieb, Glocken, Ventil oder Tunnelböden oder eine Packungskolonne mit Füllkörperpackungen.

Kopffraktion (6) wird in Kolonne (IV) in ein Kopfprodukt (8) das überwiegend gamma-Butyrolacton und 1,4-Butandiol, sowie Acetal enthält und ein Sumpfprodukt (9), das neben 1,4-Butandiol 2-Methylbutandiol, Pentandiole, Hexandiole und Heptandiole enthält, aufgetrennt. Sehr reines Butandiol wird als Produkt (10) aus dem Seitenabzug der Kolonne (IV) gewonnen. Der Seitenabzug kann dabei flüssig oder gasförmig sowohl im Verstärkungs als auch im Abtriebsteil oder genau in der Mitte der Kolonne erfolgen. Kolonne (IV) verfügt dabei über eine theoretische Bodenzahl zwischen 30 und 200, bevorzugt 50 bis 150. Der Druckbereich der Kolonne (Kopfdruck) wird bevorzugt zwischen 5 und 500 mbar (absolut) liegen. Besonders bevorzugt sind 20 bis 250 mbar. Je nach Kopfdruck und Produktzusammensetzung stellen sich die Temperaturen in der Kolonne entsprechend ein.

In einer besonderen Ausführungsform kann Kolonne (IV) eine Trennwandkolonne sein, bei der Kopffraktion (6) auf der einen Seite der Trennwand in die Kolonne eingebracht wird, während auf der anderen Seite der Trennwand reines 1,4-Butandiol (10) abgezogen wird. Weiterhin sind Kombinationen von mindestens einer, bevorzugt zwei der vorgenannten Rektifikationskolonnen mit einer Trennwandkolonne als Kolonne (IV) möglich.

Erfindungsgemäß wurde erkannt, dass für eine erfolgreiche Reinigung des 1,4-Butandiols in der Kolonne (IV), diese möglichst ohne den Eintrag von Sauerstoff betrieben wird. Sauerstoff führt in Kombination mit erhöhten Temperaturen zu Produkten, die die Reinheit des 1,4-Butandiols empfindlich stören. Diese Komponenten entstehen in Gegenwart von Sauerstoff in der Kolonne, beispielsweise im Sumpf der Kolonne, und werden als Leichtsieder in der Kolonne nach oben wandern, wobei sie automatisch ins Rein-1,4-Butandiol gelangen. Diese Komponenten sind beispielsweise gamma-Butyrolacton, 4-Hydroxybutyraldehyd oder dessen cyclisches Halbacetal oder das Acetal. Deshalb soll das molare Verhältnis Sauerstoff zu 1,4-Butandiol in der Kolonne 1 : 500 nicht übersteigt. Bevorzugt ist das Verhältnis unter 1 : 1000, besonders bevorzugt unter 1 : 1500.

Diese Verhältnisse erreicht man dadurch, dass man beim Aufbau und Betrieb der Kolonne besonders darauf achtet, keine Leckagen zu haben. Beispielsweise erreicht man das dadurch, dass Flansche verschweißt werden oder anderweitig besonders eingedichtet werden.

Die Menge des Sauerstoffs, die in die Kolonne eingetragen wird, kann beispielsweise vor Inbetriebnahme des Zulaufstromes durch Messung der Menge des Abgasstromes und dessen Sauerstoffgehaltes nach dem Vakuumaggregat in jeder der Vakuumkolonnen (III,IV V), beispielsweise durch Gaschromatographie, bestimmt werden. Während des Betriebes der Kolonne ist zu beachten, dass der Sauerstoffgehalt zu niedrig angezeigt werden könnte, da Sauerstoff unter diesen Bedingungen bereits abreagieren kann. Einen wichtigen Hinweis kann in diesem Zusammenhang das Verhältnis Sauerstoff zu Stickstoff geben, das ja dem der Umgebungsluft entsprechen sollte. Eine weitere Möglichkeit der Bestimmung des Sauerstoffgehaltes ist, die Kolonne ohne Produktzulauf zu evakuieren, die Kolonne vom Vakuumaggregat durch Schließen eines Ventils zu trennen und den Anstieg des Druckes pro Zeiteinheit in der Kolonne zu beobachten. Bei Kenntnis des Kolonnenvolumens ergibt sich daraus leicht die Menge des Sauerstoffeintrags pro Zeiteinheit.

Über Kopf von Kolonne (IV) wird ein Gemisch (8) bestehend aus überwiegend gamma-Butyrolacton mit 1,4-Butandiol und weiteren, mengenmäßig unbedeutenden Komponenten abgezogen. Dieses Kopfprodukt kann ganz oder teilweise in die Hydrierung zurückgeführt werden, wo das enthaltene gamma-Butyrolacton der pH-Regulierung dienen kann. Es ist jedoch auch möglich, das Kopfprodukt der Verbrennung zuzuführen. Bevorzugt wird dieser Kopfstrom in die Hydrierstufe zurückgeführt.

Der Sumpfstrom (9) der Kolonne (IV) enthält 1,4-Butandiol geringer Reinheit und weitere Produkte wie 2-Methyl-1,4-butandiol, Pentandiole, Hexandiole und Heptandiole sowie mengenmäßig unbedeutende Komponenten und wird ganz oder teilweise, bevorzugt ganz in Kolonne (V) geführt.

Der Sumpfstrom (9) der zweiten Kolonne wird zusammen mit dem Sumpfstrom (7) der Kolonne (III) in Kolonne (V) in ein hochsiedendes Sumpfprodukt (12), das 1,4-Butandiol, Hochsieder und Salze enthält und einen Produktstrom (11) mit 1,4-Butandiol geringer Reinheit zerlegt. Der Produktstrom (11) wird teilweise oder in Gänze verbrannt, oder bevorzugt in die Kolonne (III) zurückgeführt. Das hochsiedende Sumpfprodukt (12) kann beispielsweise in einem Fallfilm- oder Dünnschichtverdampfer bei Drücken von 0,005 bis 1 bar, bevorzugt 0,01 bis 0,7 bar, besonders bevorzugt 0,02 bis 0,4 bar in Hochsieder und schwerflüchtige organische Bestandteile wie Pentandiole, Hexandiole, Heptandiole und Salze und einen 1,4-Butandiol-enthaltenden Strom aufgetrennt werden. Dieser 1,4-Butandiol-enthaltenden Strom kann mit dem rohen 1,4-Butandiol (1), dem Produktstrom (5) oder der Sumpffraktion (7) vermischt in die erfindungsgemäße destillative Reinigung von 1,4-Butandiol zurückgeführt werden.

Erfindungsgemäß können die Vakuumanlagen mit verschiedenen Medien betrieben werden so z.B. Wasser. Es hat sich als vorteilhaft erwiesen, sie mit 1,4-Butandiol zu betreiben.

Das nach den vorstehenden Verfahrensvarianten erhaltende sehr reine 1,4-Butandiol weist üblicherweise Reinheiten von > 99,5 %, typisch sind > 99,8 % auf. Wesentliche Begleitkomponente ist dabei noch 2-Methyl-1,4-butandiol, das als Monoalkohol besonders unerwünschte Acetal liegt im Allgemeinen unter 0,1 %, im Regelfall unter 0,07 % vor.

1,4-Butandiol findet in der Technik in großen Mengen Anwendung zum Beispiel bei der THF-Herstellung oder als Diolkomponente in Polyestern.

### Beispiele

### Mengenbestimmung der Produkte

Die Analysen der Produkte wurde gaschromatographisch vorgenommen und sind bei den Reinprodukten GC-Flächen-Prozente.

### Beispiel 1

### Herstellung 1,4-Butandiol

In einer Reaktorkaskade, bestehend aus 3 zylindrischen 10 m langen Reaktoren mit einem Durchmesser von 15 cm, gefüllt mit einem Katalysator (ca. 15% CuO, ca. 4% Bi₂O3 auf SiO₂) in Form von 0,5 - 2 mm Splitt, hergestellt nach DE-A 26 02 418 , die jeweils sowohl mit Kreisgas als auch mit Flüssigumlauf in Aufwärtsfahrweise betrieben wurden (Umlauf zu Zulauf 10:1), wurden 20 kg/h 32 %iger, wässriger Formaldehyd und 2,8 kg/h Acetylen bei 5 bar und 70 bis 90°C bei einem pH von 6 umgesetzt. Das Reaktionsprodukt des ersten Reaktors wurde in den zweiten und das des zweiten in den dritten Reaktor gefördert. Auf diese Weise wurden > 95 % des Formaldehyds und des Acetylens zu 1,4-Butindiol umgesetzt. Der pH der Reaktion wurde so gesteuert, dass nach jedem Reaktor der pH gemessen wurde und bei Bedarf kleine Mengen 1 %iger wässriger NaOH-Lösung zudosiert wurden. Der Reaktionsaustrag des dritten Reaktors wurde in einem Abscheider in Gas- und Flüssigphase getrennt. Die Flüssigphase enthielt ca. 50 Gew.-% Butindiol, 1,3 Gew.-% Propinol 0,5 Gew.-% Formaldehyd, 0,5 Gew.-% Methanol, gelöstes Acetylen und mehrere 100 ppm nicht flüchtiger Oligomerer, Polymere und Katalysatorbestandteile sowie < 0,5 % andere Verunreinigungen sowie Wasser. Die Gasphase, die im wesentlichen Acetylen enthielt, wurde überwiegend als Kreisgas zurückgeführt, 1 % des Gasstrome wurde ausgeschleust. Der flüssige Austrag des Abscheiders wurde in eine Kolonne geleitet, bei der über Kopf bei 0,2 bar absolut und 90°C Sumpftemperatur Wasser, Formaldehyd, Methanol und Propinol abgetrennt (ca. 1 kg) und in die Reaktion zurückgeführt werden.

Der Sumpfaustrag wurde kontinuierlich in einen Zwischenpuffer geleitet, bei dem die mittlere Verweilzeit 10 h bei 60°C und 1 bar (absolut) betrug. Aus diesem Zwischenpuffer wurde die Butindiol-haltige Lösung entnommen und in einer zweistufigen Reaktorkaskade mit Wasserstoff an einem Ni-Katalysator gemäß EP-A 394 841 in Form von 3 x 3 mm Tabletten (ca. 38 Gew.-% Ni, ca. 12 Gew.-% Cu auf ZrO2/MoO3) hydriert. Das molare Verhältnis Frischwasserstoff zu 1,4-Butindiol betrug dabei 2,1 zu 1. Der erste Hydrierreaktor (10 m Länge, 10 cm Durchmesser) wurde mit Flüssigumlauf zur Kühlung in Aufwärtsfahrweise bei 250 bar Reaktoreingangsdruck und 120 - 140°C betrieben. Zur Einstellung des pH auf ca. 7,2 wurde dem Zulauf 1 %ige wässrige Na-OH bzw. gamma-Butyrolacton zudosiert. Der zweite Reaktor (10 m Länge, 5 cm Durchmessser) wurde in Rieselfahrweise von 140 - 160 bis 140 bis 175°C bei 250 bar betrieben. Der Austrag wurde in einem Abscheider in Flüssigphase und Gasphase getrennt, wobei die Gasphase mittels Kreisgaskompressor zurückgeführt wurde.

Im Ausgang des zweiten Reaktors wurden (wasserfrei gerechnet) ca. 94,2 % 1,4-Butandiol, 0,04 % gamma-Butyrolacton, 0,06 % 2-Methyl-1,4-butandiol, 1,6 % Methanol, 2,5 % n-Propanol, 1,2 % n-Butanol, 0,04 % Acetal sowie eine Vielzahl, mengenmäßig untergeordnete Komponenten gefunden.

### Reinigung

Anschließend wurde der entgaste Hydrieraustrag in eine Kaskade von fünf Kolonnen in die einzelnen Bestandteile aufgetrennt.
In einer ersten Kolonne (I) wurden Leichtsieder wie Methanol, Propanol und n-Butanol zusammen mit Wasser bei ca. 5 bar und einer Sumpftemperatur von ca. 170°C über Kopf abgetrennt und der Verbrennung zugeführt. Der Sumpfstrom gelangte in eine zweite Kolonne (II), bei der bei ca. 0,3 bar und ca. 130°C Sumpftemperatur ganz überwiegend Wasser über Kopf abdestilliert wurde.

Der Sumpfstrom der zweiten Kolonne (II) wurde in einer dritten Kolonne (III) bei ca. 0,15 bar und ca. 175°C Sumpftemperatur so aufgetrennt, dass über Kopf (6) überwiegend 1,4-Butandiol zusammen mit gamma-Butyrolacton, 2-Methyl-1,4-butandiol, Acetal, Pentandiolen, Hexandiolen, Heptandiolen und einigen weiteren, mengenmäßig nicht bedeutenden Komponenten abdestilliert wurde. Dieser Kopfstrom wurde in einer vierten Kolonne (IV), die bei ca. 0,04 bar und ca. 165°C Sumpftemperatur und einem Molverhältnis Sauerstoff zu 1,4-Butandiol unter 1 : 1000 betrieben wurde, in einen Kopfstrom, der neben 1,4-Butandiol überwiegend gamma-Butyrolacton sowie Acetal enthielt, einen Seitenstrom (10), der aus sehr reinem 1,4-Butandiol bestand (99,90 % 1,4-Butandiol, 0,05 % 2-Methyl-1,4-butandiol, 0,04 % Acetal) und einen Sumpfstrom, der ebenfalls aus überwiegend 1,4-Butandiol bestand und in den Sumpfstrom der dritten Kolonne (III) eingespeist wurde, aufgetrennt. Der Sumpfstrom der dritten Kolonne (III) wurde zusammen mit dem der vierten Kolonne (IV) in einer fünften Kolonne (V) bei ca. 0,05 bar und 170°C Sumpftemperatur so aufgetrennt, dass der Kopfstrom (11), der überwiegend 1,4-Butandiol enthielt, in den Zulauf der dritten Kolonne zurückgeführt wurde, während der Sumpfstrom (12), der neben wenig 1,4-Butandiol Hochsieder und Salze enthielt, ausgeschleust und verbrannt wurde.

### Vergleichsbeispiel

Beispiel 1 wurde wiederholt, mit dem Unterschied, dass das reine 1,4-Butandiol entsprechend DE-A 2 055 892 als Sumpfprodukt der Kolonne (4) gewonnen wurde. Die Reinheit dieses 1,4-Butandiol lag bei 99,75 %, 0,07 %, 2-Methyl-1,4-Butandiol, 0,04 % Acetal, 0,1 % Pentandiole, Hexandiole und Heptandiole zusammen bei 0,02 %.

## Patentansprüche

1. Verfahren zur destillativen Reinigung von rohem wasserhaltigen 1,4-Butandiol, bei dem man von leichter als 1,4-Butandiol siedenden Komponenten und Wasser befreites 1,4-Butandiol durch drei Destillationskolonnen leitet, höher als 1,4-Butandiol siedende Komponenten aus dem Sumpf der ersten Kolonne abzieht und in die dritte führt, 1,4-Butandiol vom Kopf der ersten Kolonne in die zweite führt, das Sumpfprodukt der zweiten Kolonne in die dritte führt, das Kopfprodukt der dritten Kolonne zumindest teilweise in die erste Kolonne zurückführt, wobei in der zweiten Kolonne das molare Verhältnis von Sauerstoff zu 1,4-Butandiol kleiner als 1 : 500 ist, das **dadurch gekennzeichnet ist, dass** man aus einem Seitenabzug der zweiten Kolonne das sehr reine 1,4-Butandiol entnimmt.

2. Verfahren nach Anspruch 1, **dadurch gekennzeichnet, dass** man das in der dritten Kolonne erhaltene 1,4-Butandiol zusammen mit dem Zulauf der ersten Kolonne in die Destillation zurückführt.

## Claims

1. A process for distillatively purifying crude aqueous 1,4-butanediol, in which 1,4-butanediol freed of components having lower boiling points than 1,4-butanediol and water is passed through three distillation columns, components having a higher boiling point than 1,4-butanediol are drawn off from the bottom of the first column and conducted into the third, 1,4-butanediol from the top of the first column is conducted into the second column, the bottom product of the second column is conducted into the third, the top product of the third column is recycled at least partly into the first column, the molar ratio of oxygen to 1,4-butanediol in the second column being less than 1:500, wherein the very pure 1,4-butanediol is withdrawn from a side draw of the second column.

2. The process according to claim 1, wherein the 1, 4-butanediol obtained in the third column is recycled into the distillation together with the feed of the first column.

## Revendications

1. Procédé pour la purification par distillation de 1,4-butanediol brut contenant de l'eau, dans lequel on fait passer dans trois colonnes de distillation du 1,4-butanediol libéré d'eau et de composants à plus bas point d'ébullition que le 1,4-butanediol, on soutire du pied de la première colonne les composants à plus haut point d'ébullition que le 1,4-butanediol et on les envoie dans la troisième, on envoie le 1,4-butanediol de la tête de la première colonne dans la deuxième, on envoie le produit de pied de la deuxième colonne dans la troisième, on renvoie dans la première colonne au moins en partie le produit de tête de la troisième colonne, dans la deuxième colonne le rapport molaire de l'oxygène au 1,4-butanediol étant inférieur à 1 : 500, qui est **caractérisé en ce qu'**on prélève le 1,4-butanediol très pur par un conduit de dérivation latéral de la deuxième colonne.

2. Procédé selon la revendication 1, **caractérisé en ce qu'**on renvoie dans la distillation le 1,4-butanediol obtenu dans la troisième colonne, conjointement avec le courant d'alimentation de la première colonne.
